Europäisches Patentamt

European Patent Office

Office européen des brevets

(19)

(11) Veröffentlichungsnummer : **0 328 478 B1**

## (12) EUROPÄISCHE PATENTSCHRIFT

(45) Veröffentlichungstag der Patentschrift :
22.04.92 Patentblatt 92/17

(51) Int. Cl.⁵ : **A61F 2/34**

(21) Anmeldenummer : **89730022.4**

(22) Anmeldetag : **03.02.89**

(54) Tragring für eine Hüftgelenksendoprothese.

Die Akte enthält technische Angaben, die nach dem Eingang der Anmeldung eingereicht wurden und die nicht in dieser Patentschrift enthalten sind.

(30) Priorität : **09.02.88 DE 3804310**

(43) Veröffentlichungstag der Anmeldung :
**16.08.89 Patentblatt 89/33**

(45) Bekanntmachung des Hinweises auf die Patenterteilung :
**22.04.92 Patentblatt 92/17**

(84) Benannte Vertragsstaaten :
**AT BE CH DE ES FR GB GR IT LI NL SE**

(56) Entgegenhaltungen :
**EP-A- 0 065 482**
**EP-A- 0 214 885**

(73) Patentinhaber : **MECRON MEDIZINISCHE PRODUKTE GMBH**
**Nunsdorfer Ring 23-29**
**W-1000 Berlin 48 (DE)**

(72) Erfinder : **Kranz, Curt, Dipl.-Ing.**
**Kuftsteiner Strasse 12**
**W-1000 Berlin 62 (DE)**

(74) Vertreter : **Christiansen, Henning, Dipl.-Ing.**
**Patentanwalt CHRISTIANSEN Pacelliallee 43/45**
**W-1000 Berlin 33 (DE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft einen Tragring der im Oberbegriff des Anspruchs 1 angegebenen Art.

Ein derartiger Tragring ist aus der EP 0 065 482 A2 bekannt. Diese Prothesenteile werden mit einem Schraubwerkzeug ohne Vorschneiden in die Acetabulumpfanne eingedreht und haben dort einen festen Sitz, der für eine frühzeitige Mobilität des Patienten ausreichend ist. In den Innenraum des Tragrings ist eine Pfanne aus Kunststoff einschnappbar, die als Lager für die Gelenkkugel dient.

Bei einem weiteren aus der EP 0 214 885 A1 bekannten Tragring weist nur der untere Teil gewindeförmige selbstschneidende Tragflanken auf, während der obere Teil stellenweise mit einer porösen Beschichtung versehen ist. Die primäre Verankerung des Tragrings in der Acetabulumpfanne wird durch die selbstschneidenden Tragflanken gewährleistet und die Bereiche mit poröser Beschichtung sollen das Anwachsen der Spongiosa und somit eine längerfristige gute Verankerung des Tragrings in der Acetabulumpfanne fördern.

Bei beiden bekannten Tragringen erfolgt das Einschrauben aber nur in die äußere Kortikalis-Schicht des Acetabulums.

Der Erfindung liegt die Aufgabe zugrunde, einen Tragring anzugeben, der auch bei geschädigter Kortikalis oder bei Reoperationen noch einen festen Halt findet.

Diese Aufgabe wird durch die im kennzeichnenden Teil des Anspruchs 1 angegebenen Maßnahmen gelöst.

Die Erfindung beruht auf der Erkenntnis, daß sich beim Vorsehen eines Spongiosagewindes mit entsprechend vergrößerter Gewindetiefe ein Tragflankenprofil ergibt, welches bei nach der Oberflächenkontur der Pfanne im Gewindegrund geführten Fräser eine im wesentlichen gleichbleibende Zahntiefe ergibt. Das ist auch bei der bevorzugten sphärischen Ausführung der Pfanne der Fall, welche demgegenüber sogar bei der Verwendung von Spongiosagewinde ein Einschrauben in unterschiedliche Ausrichtung ermöglicht. Damit kann auch bei dieser Tragpfanne während der Operation die optimale Ausrichtung bestimmt werden. Das gilt auch dann, wenn im Falle einer Reoperation vorher ein Tragring der vorbekannten Ausführung verwendet worden ist. Damit ist auch das neu eingeschnittene, eine größere Flankentiefe aufweisende Spongiosagewinde in der Lage, der Pfanne einen festen Halt zu geben.

Wegen der weicheren Spongiosa braucht das selbstschneidende Gewinde nicht derart ausgelegt zu werden, daß bevorzugt ausschließlich die in Einschraubrichtung vorangeführten senkrechten Schneidkanten der Tragnut spanabhebend wirken. Auch der tangentiale Rücken der Schneidflanken bewirkt ein die Gewindespur vertiefendes "Eingraben" in den Knochen.

Durch das Vorsehen von Schneidnuten unterschiedlicher Breite werden in den entsprechenden Freiräumen im Gewinde während des Schneidvorgangs Späne unterschiedlicher Größe und Menge gesammelt. Da bei einem gleichmäßigen Eingraben über die Länge des tangentialen Weges, die durch die lokale Verjüngung des Innenraumes der Pfanne in Bezug auf die entsprechende Verjüngung der Außenkontur der Schneidflanken definiert ist, die abzuhebende Spantiefe der Breite der vorangehenden Schneidflanke entspricht, werden in den durch breitere Schneidnuten vorgegebenen Bereichen zwischen den Tragflanken große Späne und in den entsprechenden Bereichen kleinerer schneidnuten, Späne geringerer Größe gesammelt.

Im Interesse eines schnellen Einheilens der Pfanne mit dem Erreichen kurzfristiger Mobilität des Patienten auch bei Reoperationen ( bei denen der Patient naturgemäß bereits ein höheres Lebensalter erreicht hat als bei der ersten Operation) wird neben der durch die Tragfähigkeit des Gewindes allein erreichten Primärfixation eine Sekundärfixation in unterschiedlichen zeitlichen Stufungen erreicht: Die sich in der schmalen Schneidnut fangenden kleineren Späne heilen verhältnismäßig schnell ein, da zwischen den dichtgepackten Spänen geringer Größe kleine Zwischenräume zu überbrücken sind. Die radial gerichteten, die "kleinen" Zwischenräume begrenzenden Kanten verhindern eine Drehbewegung der Pfanne.

In der zweiten Phase der Sekundärfixation erfolgt ein zusätzliches Einheilen durch Anwachsen der in den großen Zwischenräumen angesammelten groben Späne.

Die Sicherung in der ersten Phase der,Sekundärfixation bewirkt schon ein Blockieren sowohl in Ein-als auch in Ausschraubrichtung.

Der erfindungsgemäße Tragring weist weiterhin den Vorteil auf, daß die Tragflanken, welche nur durch eine schmale Ausnehmung (Schneidnut) voneinander getrennt sind, als Einheit tragend wirken, da die zwischen den Gewindeteilen verbleibende Spongiosa in der Lage ist, als "Brücke" zwischen diesen Tragflanken zu fungieren und die entsprechenden Kräfte aufzunehmen.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:

Figur 1 ein Ausführungsbeispiel des erfindungsgemäßen Tragrings in perspektivisher Derstellung;

Figur 2a eine erste Draufsicht auf die Tragflanken in abgewickelter Darstellung während des Einschraubvorgangs,

Figur 2b eine Schnittdarstellung zu Figur 2a sowie

Figur 2c eine zweite Draufsicht auf die Tragflanken in abgewickelter Darstellung in eingeschraubtem Zustand.

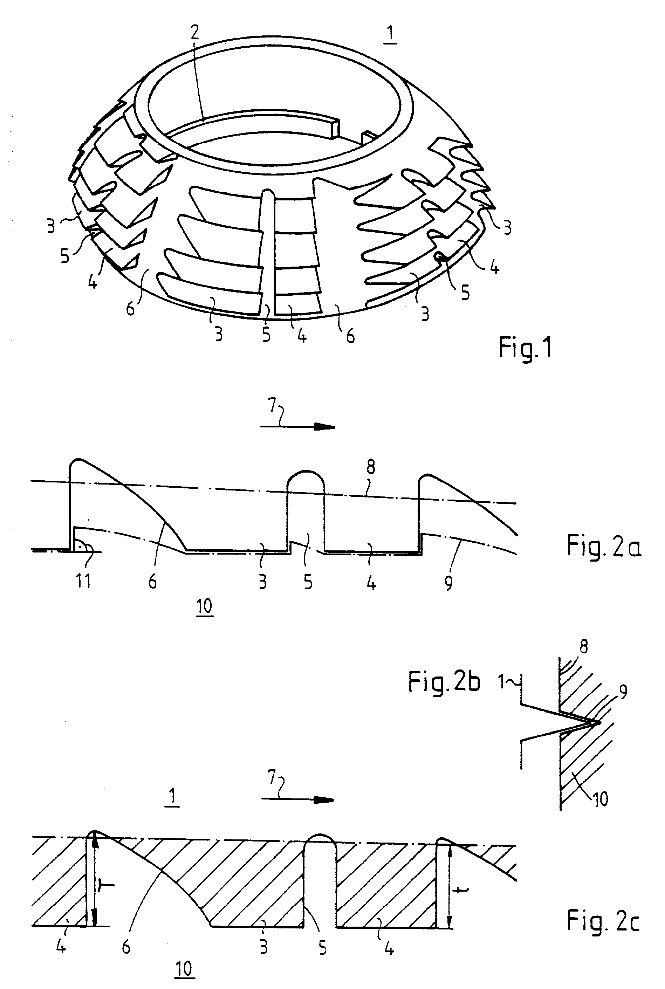

Der in Figur 1 dargestelle Tragring 1 ist aus Titan gefertigt und dient zur Aufnahme einer Kunststoff-Gelenkpfanne, die das Gelenklager bildet. An der Innenwandung vorgesehene Vorsprünge 2 bilden ein Widerlager für den Halt der Kunststoff-Gelenk-Pfanne.

Die Außenwandung des Tragrings ist sphärisch ausgebildet (bezogen auf den Gewindegrund), so daß der in unterschiedlichen Richtungen in ein ebenfalls sphärisch ausgefrästes Acetabulum einschraubbar ist, ohne daß es weiterer Vorbereitungen, wie Gewinde schneiden, bedarf.

An der Außenseite sind Gruppen von Tragflanken vorgesehen, welche neben ihrer unterschiedlichen Ausformung jeweils reihenweise mit 3 oder 4 bezeichnet sind. Die Tragflanken wurden erzeugt mit einem Fräser für Spongiosaprofil, das für Knochenschrauben in verschiedenen Ausführungen nach DIN 17443 genormt ist. Der Fräser bewegt sich dabei in axialer Richtung auf der Kontur des sphärischen Gewindesgrundes. Im Fall der geometrischen Verhältnisse verändert sich die Form der Tragflanken innerhalb einer Gruppe 3 bzw. 4 in axialer Richtung geringfügig. Sie behalten jedoch (bis auf die obersten Flanken jeder Gruppe im Bereich des Auslaufbereichs des Gewindes) ihre Größe im wesentlichen bei, so daß sie gleichmäßig zur Überleitung der auftretenden Belastungen in den Knochen beitragen können.

Zwischen den Gruppen von Tragflanken 3 bzw. 4 sind abwechselnd schmale und verbreitete Schneidnuten 5 bzw. 6 eingefräst, wobei diese Schneidnuten mit einem weiteren Fräser jeweils entsprechenden Profils nach Erzeugung des Gewindes durch Führung auf dem Teil einer Kreisbahn erzeugt wird.

Die schmalen Schneidnuten 5 sind seitlich durch radial gerichtete Ebenen begrenzt, während ihr Grund verrundet ist. Die breiteren Schneidnuten 6 sind zur einen Seite (in Schneidrichtung vorangeführte Kanten der Tragflanken) durch eine radial gerichtete Ebene begrenzt, während die andere Seite abgeschrägt ist, so daß das Profil in der Draufsicht einen im wesentlichen dreiecksförmigen Ausschnitt zeigt, wobei ein rechter Winkel des Dreiecks (in der abgewickelten Darstellung gemäß Figuren 2a bzw. 2c) zwischen der gedachten Fortsetzung der Außenkante der Tragflanken und der beim Schneiden vorangeführten Kante der Trägflankengruppe 4 gebildet wird. Ein derartiger rechter Winkel 11 ist in Figur 2a mit einem gestrichelt dargestellten Schenkel wiedergegeben.

In Figur 2a ist das Tragflankenprofil vergrößert in der Draufsicht wiedergegeben, wobei die Zeichnung eine Abwicklung darstellt bei der der Gewindegrund geradlinig verläuft. Die Einschraubrichtung ist durch den Pfeil 7 symbolisiert.

Die Gerade 8 stellt in der Abwicklung die Kante der Innenfläche des Acetabulums dar, welche sich entgegen der Einschraubrichtung dem Gewindegrund der Tragflanken (gestrichelte Linie 9) annähert, da sich deren Abstand entgegengesetzt zur Einschraubrichtung 7 verengt.

Die Linie 9 bildet den Grund der in das Acetabulum 10 eingeschnittenen Gewindespur (die Verhältnisse sind in Figur 2b als Schnittdarstellung zu Figur 2a im Detail ersichtlich).

Der Verlauf der Kante 9, die ursprünglich zu der Linie 8 parallel verläuft, zeigt, daß ein Einschneiden in das Acetabulum sowohl durch die senkrecht gerichteten Vorderkanten der Tragflankengruppe 4 als auch durch die entsprechenden Kanten der Tragflankengruppe 3 vorgenommen wird. Da die Vertiefung durch die Gruppe 4 größer ausfällt als diejenige der Gruppe 3, bilden sich hier größere Späne, die von dem entsprechend vergrößerten durch die Schneidnuten 6 gebildeten Raum aufgenommen werden.

Da der Tragring 1 sich relativ zum Acetabulum auf der Linie 8 bewegt, drücken sich auch noch die tangentialen Kanten der Tragflanken in das Acetabulum ein, da diese Kanten nicht entsprechend abgeschrägt sind. Wegen der verhältnismäßig geringen Festigkeit der Spongiosa wird durch die damit verbundene Kompression jedoch lediglich eine gewisse Materialverdichtung erzeugt. Gerade die verdichteten Bereiche werden aber durch die Schneidkante der nachfolgenden Tragflanke beseitigt und als Span in den durch die Schneidnuten gebildeten Hohlraum eingebracht.

Aus der Draufsicht der weiteren Figur 2c ist die Tragwirkung der Flanken 3 und 4, die mit einer Einschrabtiefe t wollständig im Acetabulum 10 einpeschraubt sind, erkennbar. Die Druckkräfte aufnehmenden Bereiche der Tragflankengruppen 3 und 4 sind relativ großflächig, wobei die Tiefe T der tragflänken 3 und 4 zwischen 4 und 6 mm beträgt. Die Breite der Nut 5 (zwischen 1 und 2 mm) bildet keine wesentliche Unterbrechung für die Krafteinleitung und wird von der Spongiosa überbrückt. Knochenmaterial, das sich in dieser Nut ansammelt, bildet eine Verriegelung gegen Bewegungen in Richtung des Pfeils 7 als auch entgegengesetzt dazu. In dem durch die Nut 6 gebildeten Bereich können sich dagegen größere Späne ansammeln, welche langfristig für einen stabilen Sitz der Prothese sorgen.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch machen.

**Patentansprüche**

1. Tragring für eine Hüftgelenksendoprothese mit selbstschneidenden Gewindetragflanken mit Knochenschraubenprofil und zwischen den Tragflanken angeordneten Schneidnuten, **dadurch gekennzeichnet**, daß das Knochenschraubenprofil als Spongiosa-Gewindeprofil ausgebildet ist und daß Schneidnuten (5, 6) unterschiedlicher Breite vorgesehen sind, wobei die Breite der einen Schneidnuten (5) geringer ist als die Tiefe (T) der Tragflanken (3, 4) und die Breite der anderen Schneidnuten (6) größer ist als die Tiefe (T) der Tragflanken (3, 4).

2. Tragring nach Anspruch 1, **dadurch gekennzeichnet**, daß die Abmessungen der Tragflanken (3, 4) in tangentialer Erstreckung im Bereich des maximalen Radius dem 1,0- bis 1,5-fachen von deren Tiefe entsprechen.

3. Tragring nach einem der vorangehenden Ansprüche, **dadurch gekennzeichnet**, daß die Tiefe (T) der Tragflanken (3, 4) zwischen 4 und 6 mm ist.

4. Tragring nach einem der vorangehenden Ansprüche, **dadurch gekennzeichnet**, daß die schmalen Schneidnuten (5) zwischen 1 und 2 mm breit sind.

5. Tragring nach einem der vorangehenden Ansprüche, **dadurch gekennzeichnet**, daß die Rückflanken der an die breiten Schneidnuten (6) angrenzenden Kanten in der Weise abgeschrägt sind, daß sie sich in der Draufsicht mit zunehmendem radialen Abstand verjüngen.

6. Tragring nach einem der vorangehenden Ansprüche, **dadurch gekennzeichnet**, daß die schmalen Schneidnuten (5) zur Mittelachse des Rings innen hin verrundet sind.

7. Tragring nach einem der vorangehenden Ansprüche, **dadurch gekennzeichnet**, daß die Außenoberfläche, bezogen auf den Gewindegrund, sphärisch ausgebildet ist.

**Claims**

1. Support ring for a hip joint endoprosthesis with self-tapping thread support flanks of bone screw profile, and with cutting grooves arranged between the support flanks, characterised in that the bone screw profile is designed as a spongiosa thread profile, and in that cutting grooves (5, 6) of different width are provided, the width of the first cutting grooves (5) being smaller than the depth (T) of the support flanks (3, 4), and the width of the second cutting grooves (6) being greater than the depth (T) of the support flanks (3, 4).

2. Support ring according to Claim 1, characterised in that the dimensions of the support flanks (3, 4) in the tangential extent in the area of the maximum radius correspond to 1.0 to 1.5 times their depth.

3. Support ring according to one of the preceding claims, characterised in that the depth (T) of the support flanks (3, 4) is between 4 and 6 mm.

4. Support ring according to one of the preceding claims, characterised in that the narrow cutting grooves (5) are between 1 and 2 mm wide.

5. Support ring according to one of the preceding claims, characterised in that the back flanks of the edges adjacent to the wide cutting grooves (6) are bevelled in such a way that, in the plan view, they taper with an increasing radial distance.

6. Support ring according to one of the preceding claims, characterised in that the narrow cutting grooves (5) are rounded in towards the centre axis of the ring.

7. Support ring according to one of the preceding claims, characterised in that the outer surface, relative to the thread base, is of spherical design.

**Revendications**

1. Anneau de support pour une endoprothèse de la hanche présentant des flancs de support filetés autotaraudeurs avec profil hélicoïdal d'os et des rainures de coupe disposées entre les flancs de support, caractérisé en ce que le profil hélicoïdal d'os est conçu comme un profil fileté de tissu spongieux et en ce qu'il est prévu des rainures de coupe (5, 6) de largeur différente, la largeur d'une rainure de coupe (5) étant inférieure à la profondeur (T) des flancs de support (3, 4) et la largeur des autres rainures de coupe (6) étant supérieure à la profondeur (T) des flancs de support (3, 4).

2. Anneau de support selon la revendication 1, caractérisé en ce que les dimensions des flancs de support (3, 4) dans la direction tangentielle, dans la région du rayon maximum, sont comprises entre 1 et 1,5 fois leur profondeur.

3. Anneau de support selon l'une des revendications précédentes, caractérisé en ce que la profondeur (T) des flancs de support (3, 4) est comprise entre 4 et 6 mm.

4. Anneau de support selon l'une des revendications précédentes, caractérisé en ce que les rainures de coupe (5) étroites ont une largeur comprise entre 1 et 2 mm.

5. Anneau de support selon l'une des revendications précédentes, caractérisé en ce que les flancs arrière des bords adjacents aux rainures de coupe (6) larges, sont chanfreinés de manière à se rétrécir, en vue de dessus, au fur et à mesure que la distance radiale augmente.

6. Anneau de support selon l'une des revendications précédentes, caractérisé en ce que les rainures de coupe (5) étroites sont arrondies vers l'intérieur, en direction de l'axe médian de l'anneau.

7. Anneau de support selon l'une des revendications précédentes, caractérisé en ce que la surface extérieure est sphérique par rapport au fond du filetage.

Fig.1

Fig.2a

Fig.2b

Fig.2c